# EUROPEAN PATENT SPECIFICATION

(11) **EP 2 473 220 B1**
(45) Date of publication and mention of the grant of the patent: **07.11.2018**
(21) Application number: 10814608.5
(22) Date of filing: 03.09.2010
(51) Int. Cl.: A61M 16/12, A61M 16/06

(54) **SYSTEM FOR NON-INVASIVE VENTILATION INCLUDING A NON-SEALING VENTILATION INTERFACE WITH A FREE SPACE NOZZLE FEATURE**
SYSTEM FÜR NICHT INVASIVE BEATMUNG MIT EINEM NICHT ABDICHTENDEN BEATMUNGSINTERFACE MIT FREIRAUMDÜSENFUNKTION
SYSTÈME NON EFFRACTIF DE VENTILATION NASALE COMPRENANT UNE INTERFACE NON FERMÉE AVEC ESPACE LIBRE POUR L' INJECTEUR

(30) Priority: 03.09.2009 US 239728 P; 28.10.2009 US 255760 P; 12.01.2010 US 294363 P; 19.02.2010 US 306370 P; 02.04.2010 US 753846; 02.04.2010 WO PCT/US2010/029871; 02.04.2010 US 753851; 02.04.2010 WO PCT/US2010/029873; 02.04.2010 US 753853; 02.04.2010 US 753854; 02.04.2010 WO PCT/US2010/029874; 02.04.2010 US 753856; 02.04.2010 WO PCT/US2010/029875
(43) Date of publication of application: 11.07.2012
(73) Proprietor: Breathe Technologies, Inc., San Ramon, CA 94583 (US)
(72) Inventor: CIPOLLONE, Joseph, San Ramon California 94583 (US); AGUIRRE, Joey, San Ramon California 94583 (US); ALLUM, Todd, Livermore California 94551 (US); EGHBAL, Darius, Oakland California 94609 (US); WONDKA, Anthony D., Thousand Oaks California 91362 (US)
(74) Representative: MacDougall, Alan John Shaw
(86) International application number: PCT/US2010/047920
(87) International publication number: WO 2011/029073

(56) References cited:
- WO-A1-03/068301
- WO-A2-2005/007056
- WO-A2-2005/011556
- US-A1- 2005 011 524
- US-A1- 2005 011 524
- US-A1- 2005 121 033
- US-A1- 2007 074 724
- US-A1- 2007 074 724
- US-A1- 2007 095 347
- US-A1- 2007 095 347

## Description

### FIELD OF THE INVENTION

The present invention relates to the field of ventilation therapy for persons suffering from respiratory and breathing disorders, such as respiratory insufficiency and sleep apnea. More specifically, the present invention relates to providing open airway ventilation with devices that use non-sealing non-invasive nasal ventilation patient interfaces.

### BACKGROUND OF INVENTION

There is a need for a minimally obtrusive nasal mask and ventilation system that delivers mechanical ventilatory support or positive airway pressure, and which unencumbers the patient. There are a range of clinical syndromes that require ventilation therapy that would benefit from such a mask and system, such as respiratory insufficiency, airway or sleeping disorders, congestive heart failure, neuromuscular disease, and a range of situations that would be benefited, such as chronic, acute, emergency, mass casualty and pandemic situations.

Oxygen therapy is available with devices that do not encumber the patient. However, oxygen therapy is used for far less severe forms of clinical syndromes compared to ventilation therapy. For example, some nasal mask oxygen therapy systems have been developed for the purpose of delivering mixtures of air and oxygen by entraining air into the mask, however these are not considered ventilation therapy or respiratory support, because they do not mechanically help in the work of breathing. Recently, a variant of oxygen therapy has been employed, known as high flow oxygen therapy (HFOT). In this case, the oxygen flow rate is increased beyond standard long term oxygen therapy (LTOT), for example, above 15 LPM. Because of the high flow rate, the oxygen must be humidified to prevent drying out the patient's airway. It has been reported that HFOT can slightly reduce the patient's absolute pleural pressure during spontaneous breathing, thus have a slight effect on work of breathing. These systems are inefficient in that they consume a significant quantity of oxygen, rendering them non-mobile systems and encumbering the patient.

Respiratory support and ventilation therapies exist that provide mechanical ventilation (MV) to the patient, and mechanically contribute to the work of breathing. MV therapies connect to the patient by intubating the patient with a cuffed or uncuffed tracheal tube, or a sealing face or nasal mask or sealing nasal cannula. While helpful in supporting the work of breathing, the patient interfaces used for MV are obtrusive and/or invasive to the user, and MV does not facilitate mobility or activities of daily living, therefore encumbers that patient and is a drawback to many potential users. Noninvasive ventilation (NIV) exists which ventilates a patient with a face or nasal mask rather than requiring intubation, which can be an advantage in many situations. However, the patient cannot use their upper airway because the interface makes an external seal against the nose and/or mouth, and in addition the system is not mobile, the combination of which does not enable activities of daily living.

For treating obstructive sleep apnea (OSA), the gold standard ventilation therapy is continuous positive airway pressure (CPAP) or bilevel positive airway pressure (BiPAP), which is a variant to NIV in that the patient partially exhales through exhaust ports in the mask and back into large gas delivery tubing, rather than through an exhalation circuit as in MV. Continuous positive pressure applied by the ventilator to the patient by a nasal or face mask that seals against the nose or face prevents upper airway obstruction. While effective, this therapy has poor patient compliance because the patient interface is obtrusive to the patient and the patient unnaturally breathes through both a mask and gas delivery circuit.

In summary, existing therapies and prior art have the following disadvantages: they do not offer respiratory support or airway support in a manner that unencumbers the patient and (1) is non-invasive, and un-obtrusive such that it allows for mobility and activities of daily living, (2) allows the sensation of breathing from the ambient surroundings normally, and (3) is provided in an easily portable system or a system that can be easily borne or worn by the patient.

US2007/095347 discloses an apparatus and method for the delivery of inspired gas, e.g., supplemental O₂, to a person combined with gas sampling, including for the purpose of monitoring of the ventilation of the person. According to one embodiment, an oronasal piece is described, intended for use with a gas delivery and gas sampling system. A pressure sampling lumen and a gas sampling lumen are contained within a left nostril insert that fits into the left nare of the patient. A pressure sampling lumen and a gas sampling lumen are contained within a right nostril insert that fits into the right nare of the patient. A multiplicity of holes diffuse O₂ near the region of the nares.

WO03/068301 discloses a compact nasal or nasal/oral cannula comprising separate gas supply and gas collection lumens having a common wall. The cannula provides dual functionality of gas collection and supply, applied equally to both nostrils of a subject. The lumens for the collection and supply functions of the cannula have different dimensions and may be embedded, one partially within the other.

### SUMMARY OF INVENTION

The present invention is a system for supplying ventilatory support as defined in Claim 1 of the appended claims.

The invention provides ventilation to a patient using non-invasive open-airway ventilation (NIOV), and a non-sealing nasal mask interface with nozzles in free space that does not completely cover or seal the opening of the patient's mouth or nose.

According to an aspect of the present invention there is provided a system for supplying ventilatory support, the system comprising: a gas delivery source; a gas delivery circuit; a non-sealing nasal interface configured to communicate with a patient's nose while allowing the patient to breathe ambient air directly without flowing through the non-sealing nasal interface; a nozzle associated with the non-sealing nasal interface at a distance from the patient's nose, wherein the nozzle is connectable to the gas delivery circuit and the gas delivery source; and wherein the nozzle is capable of delivering gas into the nasal passage by creating a negative pressure area near the nozzle and a positive pressure area near the entrance to the patient's nose, wherein a combination of gas from the gas delivery source and air entrained from the gas exiting the nozzle provide ventilatory support.

The present disclosure describes a method for providing ventilatory support, the method including: providing a nasal interface that allows the patient to breathe ambient air through the nasal interface; providing a nozzle in free space associated with a proximal end of the nasal interface at a distance from a nose; adapting the nozzle to be in fluid communication with a gas delivery circuit and a gas delivery source, wherein the nozzle is capable of delivering gas into the nasal interface to create a negative pressure area near the nozzle and a positive pressure area near the entrance to the nose, and wherein a combination of gas from the gas delivery source and air entrained by the nozzle provides ventilatory support.

The nozzle may be capable of creating the positive pressure area at a point outside the nose and distal to that point. The positive pressure area may be created at an edge of a nostril rim and distal to the edge. The positive pressure area may be created at a point in a nostril airway and distal to that point. The non-sealing nasal interface may comprise a manifold, and wherein the manifold comprises the nozzle, and wherein the manifold is configured to position the nozzle at a distance away from a nostril entrance, and configured to position the nozzle at an angle relative to a centerline of a nostril airway. The system may include one or more sensors, wherein the one or more sensors comprise a sensing channel that extends away from the nozzle toward the nose terminating in the positive pressure area. The system may include one or more sensors, wherein the one or more sensors comprise a sensing channel that extends distally away from the nozzle. The sensing channel may be configured to extend into the patient's nose during use. The sensing channel may extend to within approximately +/- 5 mm from a nostril entrance. The system may include two or more nozzles per nostril. The nozzle may be an oval-shaped gas delivery nozzle orifice. The nozzle may include an array of multiple gas delivery nozzles arranged in a pattern to approximate the shape of the patient's nostril. The system may include a jet pump throat including a flow path. The jet pump throat may be associated with a manifold, and the nozzle may be associated with a jet pump throat flow path through the jet pump throat. The manifold may include an entrainment port in communication with the jet pump throat flow path. The nozzle may angle inward relative to an axis perpendicular to a longitudinal axis of the non-sealing nasal interface. The nozzle may angle inward at an angle of approximately 1 - 20 degrees. The nozzle may create a gas delivery flow profile shaped to approximate the shape of a patient's nasal opening. The nozzle may be rotatably adjustable. The nozzle may include at least one left nozzle and at least one right nozzle, wherein the spacing between the at least one left nozzle and the at least one right nozzle is adjustable. The at least one left nozzle and the at least one right nozzle may be rotate-ably adjustable. Spacing between a nostril entrance and nozzle may be adjustable. The nasal interface may be available in different sizes, differing in nozzle spacing, nozzle rotational orientation and nozzle distance to nostril entrance. The negative pressure area may extend from the nozzle to a location proximal to an entrance to a nose. A negative pressure may be less than ambient. The negative pressure may be approximately -5 to -28 cmH₂0. The positive pressure area may extend from a location distal to the nozzle to an entrance to a nose. The positive pressure may be greater than ambient. The system may be configured to provide a positive pressure of approximately 0.7 - 34.5 mBar (0.01 - 0.50 psi). The combination of gas from the gas delivery source and the air entrained through entrained from the gas exiting the nozzle may be laminar flow within a nose. The system may be configured to allow the nozzle to be positioned approximately 0 - 3.8 cm (0 - 1.5 inch) outside the entrance to the patient's nose. The system may be configured to allow delivery of gas through the nozzle to be synchronized with a breathing pattern of the patient. The system may further comprise a wearable ventilator, wherein the gas from the gas delivery source is controlled by the wearable ventilator. Ventilatory support may include reducing the work of breathing to treat respiratory insufficiency. Ventilatory support may include elevating airway pressure to treat sleep apnea. The nasal interface may include a connector for coupling the system to a bridge of the nose and aligning the at least one gas delivery jet nozzle with the entrance of the nose. The connector may include a ledge to position the nasal interface relative to an edge of a nostril rim. The connector may adjust the angle of the nozzle to be in alignment with a centerline of a nostril airway.

The present disclosure also describes a system for supplying ventilatory support, the system including: a gas delivery source; a gas delivery circuit; a nasal interface configured to communicate with a patient's nose while allowing the patient to breathe ambient air directly without flowing through the nasal interface; a nozzle associated with the nasal interface at a distance from a nose, wherein the nozzle is connectable to the gas delivery circuit and the gas delivery source; a jet pump throat comprising a flow path through the jet pump throat, wherein the jet pump throat is associated with a manifold, and the nozzle is associated with a jet pump throat flow path through the jet pump throat; and an entrainment port in communication with the jet pump throat flow path, wherein the nozzle is capable of delivering gas into the nasal passage by creating negative pressure area near the nozzle within the jet pump throat flow path and a positive pressure area within the jet pump throat flow path distal to the nozzle, wherein a combination of gas from the gas delivery source and air entrained through the entrainment port provide ventilatory support. Ventilatory support may mechanically contribute to reducing the work of breathing to treat respiratory insufficiency. Ventilatory support may include elevating airway pressure to treat sleep apnea.

Additional features, advantages, and embodiments of the invention are set forth or apparent from consideration of the following detailed description, drawings and claims. Moreover, it is to be understood that both the foregoing summary of the invention and the following detailed description are exemplary and intended to provide further explanation without limiting the scope of the invention as claimed.

### BRIEF DESCRIPTION OF THE DRAWINGS

The accompanying drawings, which are included to provide a further understanding of the invention and are incorporated in and constitute a part of this specification, illustrate preferred embodiments of the invention and together with the detailed description serve to explain the principles of the invention.
Figure 1 shows a prior art conventional oxygen delivery cannula for administering oxygen therapy.
Figure 2 shows a prior art conventional non-invasive ventilation using a nose mask and using a CPAP or BiPAP ventilation mode.
Figure 3 shows an unencumbered patient using an embodiment of the invention to receive work of breathing support while ambulating.
Figure 4 is a schematic showing an exemplary system of the invention.
Figure 5 shows an exemplary embodiment where an open non-sealing nasal ventilation mask is configured to be placed under the nose of the user, and which may extend bilaterally from the midline of the face to the sides of the nose.
Figure 6 is a perspective view of the nasal mask assembly of Figure 5.
Figure 7 shows a front view schematic illustration of an embodiment of the nasal mask.
Figure 8 shows an anterior-top-side view of the nasal mask of Figure 5.
Figure 9 shows a hidden line view of the nasal mask of Figure 8, showing the gas flow path and breathing pressure sensing path.
Figure 10 shows a top view of the nasal mask of Figure 5.
Figure 11 shows a hidden line view of the nasal mask of Figure 10, showing the gas flow path and breathing pressure sensing path.
Figure 12 shows a rear-top view of the nasal mask of Figure 5.
Figure 13 shows a hidden line view of the nasal mask of Figure 12, showing the gas flow path and breathing pressure sensing path.
Figure 14 shows a pattern created by flow emission from gas delivery nozzles.
Figure 15 shows a pattern created by flow emission from gas delivery nozzles.
Figure 16 shows an embodiment of the nasal mask shown in Figure 5 with oval gas delivery nozzles.
Figure 17 shows an embodiment of the nasal mask shown in Figure 5 with multiple gas delivery nozzles arranged in an anatomically functional pattern.
Figure 18 shows a hidden line view of the mask view of an embodiment of the nasal mask, showing the gas flow path and breathing pressure sensing path, in which the gas delivery nozzles are angled inward toward the midline.
Figure 19 shows an embodiment of the mask shown in Figure 5 with a jet pump throat with a Venturi inlet at the bottom of the mask manifold.
Figure 20 shows a mask worn by a user with a jet pump throat with a Venturi inlet at the top of the manifold near the base of the throat.
Figure 21 shows an alternative embodiment of the nasal mask where the gas delivery nozzles are positioned under the nose by a nose piece with extension arm.
Figure 22 shows the pressure sensing and gas delivery flow patterns of the nasal mask of Figure 21 in the nostril airway.
Figure 23 shows the mask assembly of the nasal mask shown in Figure 21.
Figure 24 shows an alternate embodiment of the nasal mask shown in Figure 21 in which the gas delivery nozzles are positioned under the nose with an extended nose piece.
Figure 25 shows an embodiment of the nasal mask shown in Figure 21 with a minimized nose piece and streamlined vertical and horizontal arms.
Figure 26 shows an embodiment of the nasal mask shown in Figure 21 in which the gas delivery nozzles are positioned under the nose by a head gear and bracket.
Figure 27 graphically shows how the patient's work of breathing may be beneficially affected by the invention when the invention is used for lung disease or neuromuscular disease applications.
Figure 28 graphically shows lung volume on the x-axis and lung pressure on the y-axis to illustrate how the lung volumes achieved with NIOV on a lung simulator bench model in comparison to conventional ventilation.
Figure 29 graphically shows the lung volumes achieved with NIOV in comparison to oxygen therapy, using the lung simulator bench model.
Figure 30A graphically shows a square waveform gas delivery pressure, according to one embodiment.
Figure 30B graphically shows the volume delivery of Figure 30A.
Figure 30C graphically shows resulting lung pressure of Figure 30A.
Figure 30D graphically shows a sinusoidal waveform gas delivery pressure, according to one embodiment.
Figure 30E graphically shows the volume delivery of Figure 30D.
Figure 30F graphically shows resulting lung pressure of Figure 30D.
Figure 30G graphically shows a square waveform gas delivery pressure for a portion of the inspiratory phase, according to one embodiment.
Figure 30H graphically shows the volume delivery of Figure 30G.
Figure 30I graphically shows resulting lung pressure of Figure 30G.
Figure 30J graphically shows a multi-level waveform gas delivery pressure, according to one embodiment.
Figure 30K graphically shows the volume delivery of Figure 30J.
Figure 30L graphically shows resulting lung pressure of Figure 30J.
Figure 31A graphically shows an ascending waveform gas delivery pressure, according to one embodiment.
Figure 31B graphically shows the volume delivery of Figure 31A.
Figure 31C graphically shows resulting lung pressure of Figure 31A.
Figure 31D graphically shows a descending waveform gas delivery pressure, according to one embodiment.
Figure 31E graphically shows the volume delivery of Figure 31D.
Figure 31F graphically shows resulting lung pressure of Figure 31D.
Figure 31G graphically shows a two-stage amplitude waveform gas delivery pressure for a portion of the inspiratory phase, according to one embodiment.
Figure 31H graphically shows the volume delivery of Figure 31G.
Figure 311 graphically shows resulting lung pressure of Figure 31G.

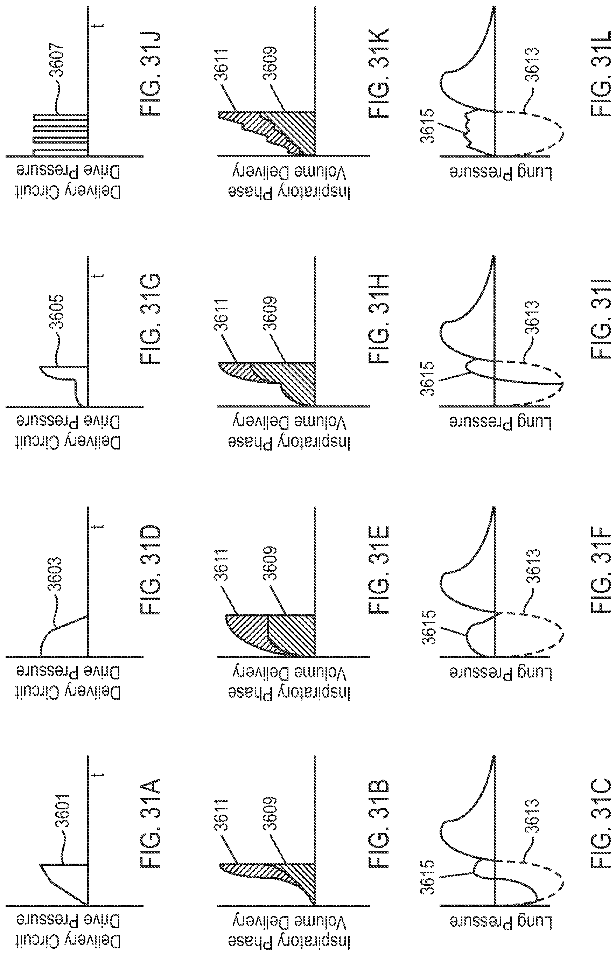

Figure 31J graphically shows an oscillatory waveform gas delivery pressure, according to one embodiment.
Figure 31K graphically shows the volume delivery of Figure 31J.
Figure 31L graphically shows resulting lung pressure of Figure 31J.

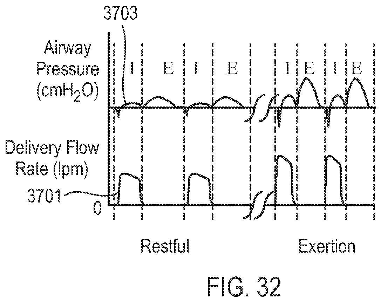

Figure 32 graphically shows the timing and amplitude of a breath frequency modulated gas flow amplitude delivery, according to one embodiment.

### DETAILED DESCRIPTION OF THE EMBODIMENTS

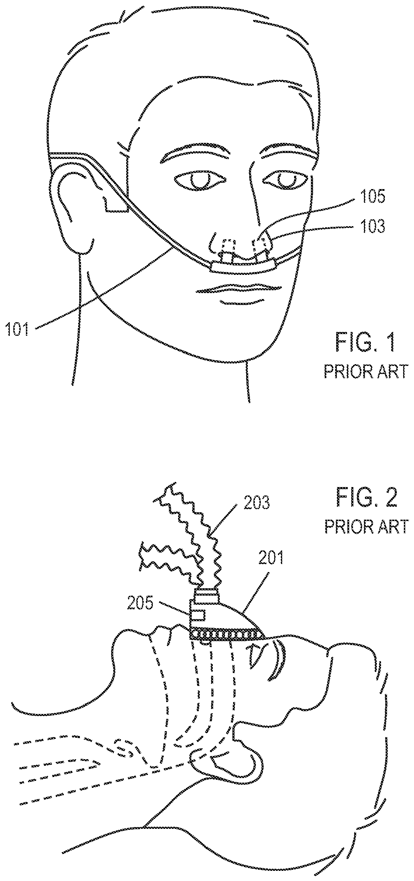

Figure 1 shows a prior art conventional oxygen delivery cannula 101 for administering oxygen therapy. Extensions 105 on the cannula 101 are configured to enter nares 103. A proximal end (not shown) of the cannula 101 is connected to an oxygen delivery device that delivers continuous flow oxygen at 1-6 LPM to the user's nose, or delivers a bolus of oxygen upon detection of an inspiratory effort. The system of Figure 1 does not mechanically support the work of breathing of the patient, and is not believed to be effective in preventing moderate to severe forms of OSA. The cannula of Figure 1 is also used with another oxygen delivery therapy, high flow oxygen therapy (HFOT), in which more than 15 LPM of humidified oxygen is delivered at a continuous flow rate to the user's nose. Due to the high flow required for HFOT, the system is non-portable and the oxygen must be humidified.

Figure 2 shows a prior art respiratory support therapy for non-invasive ventilation (NIV), using a nose mask 201 in a bilevel positive airway pressure (BiPAP) ventilation mode. NIV is used to breathe for the patient, or can be used to help the breathing of a patient, in which case the patient's spontaneous breathing effort triggers the ventilator to deliver the pressure or volume-based mechanical ventilation (MV). All of the volume delivered to and from the lungs is delivered and removed from a ventilation circuit 203 and the nose mask 201.

A similar system to Figure 2 can be used for OSA where a mask is sealed to the face so ventilation gas is provided by the ventilator and a portion of exhaled gas is exhaled through exhaust vents 205. NIV, continuous positive airway pressure (CPAP) and BiPAP are believed to be clinically effective modes and therapies for spontaneously breathing patients. These modes and therapies, however, do not facilitate activities of daily living (ADL's). For example, the ventilator cannot be borne by the patient, the patient cannot breathe room air naturally and freely because of the sealing mask, and the patient's upper airway cannot function normally and naturally because it is sealed off with the external mask seal, and in addition the gas delivery tubing is too bulky to realistically support mobility and ADL's .

Embodiments of the present invention will now be described with reference to the remaining figures. Respiratory support or airway support is provided in a manner and way that the patient is unencumbered. The non-invasive, non-sealing and unobtrusive systems and methods may allow for mobility and activities of daily life. The systems and methods allow for the sensation of breathing from ambient surroundings normally. The systems and methods provide an easily portable system that can be readily borne or worn by the patient, and gas delivery tubing that does not encumber the patient.

Systems and methods may include a gas delivery source, a gas delivery circuit, and a nasal interface that allow breathing ambient air through the nasal interface. A gas flow path through the nasal interface may have a distal gas flow path opening. A nozzle may be associated with a proximal end of the nasal interface a distance from the distal end gas flow path opening. In certain embodiments, at least a portion of an entrainment port may be between the nozzle and the distal end gas flow opening. The nozzle may deliver gas into the nasal interface to create a negative pressure area in the gas flow path at the entrainment port. The nasal interface and the nozzle may create a positive pressure area between the entrainment port and the distal end of the nasal interface. Gas from the gas delivery source and air entrained through the entrainment port may increase airway pressure.

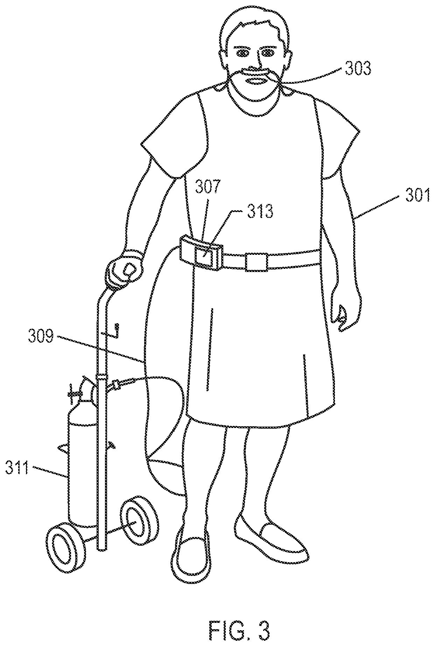

Figure 3 shows a patient 301 using an embodiment of the invention to provide mechanical ventilatory support, or work of breathing support, while being mobile. Conventional ventilators would require the patient to be stationary while receiving ventilatory support, or to use a wheel chair to carry the bulky and heavy equipment that is required for conventional ventilators. Conventional ventilators also require an encumbering sealing mask and large bore gas delivery tubing. The patient may also wear a ventilator module 307, which may be ultra-small that enables mobility when the invention is used for respiratory insufficiency. The ventilator may be coupled by tubing or other means 309 to an air and or oxygen supply 311. The ventilator module 307 may include a display 313 and/or input devices.

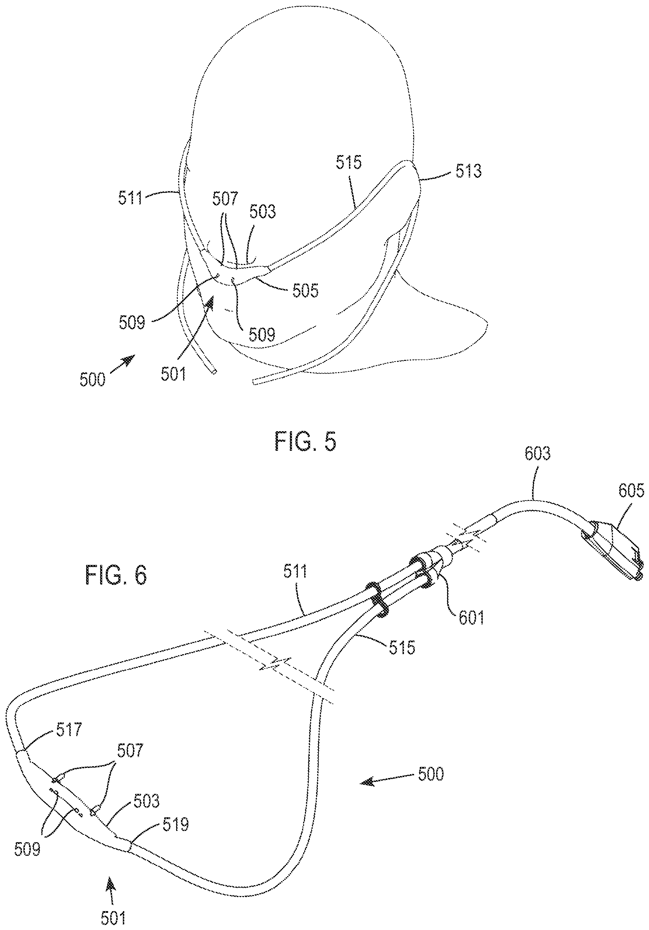

The present invention may include a non-sealing nasal mask patient interface, connected to the ventilator with small bore gas delivery tubing. The nasal mask may be uniquely non-sealing, so that the patient can inhale and exhale ambient air directly through the mask while receiving ventilatory support, in which there is negligible dead space volume in the mask. The mask may include a unique Venturi system that makes it possible for the ventilator to deliver relatively small amounts of gas to achieve relatively high levels of ventilatory support or airway pressure. The Venturi mask is described in more detail in Figures 6 - 31.

Various embodiments of the nasal interface 303 are described in detail in the following disclosure. The nasal interface 303 may be minimally obtrusive compared to standard masks, so that the patient can feel and act normally while receiving the therapy. For example, the patient can talk, swallow, eat or drink, and feel like they are breathing normally, with the nasal interface and therapy. The gas delivery tubing required may be very small compared to standard ventilator tubing, which more readily allows the patient to move around with the system, and to conceal the equipment and tubing needed for the therapy. The efficiency of the Venturi system in achieving therapeutic levels of lung or airway pressure while using low levels of gas volume, allows the gas supply to be relatively small, further enabling mobility of the patient, and or miniaturization of the ventilation equipment. A nasal interface may be configured to communicate with a patient's nose while allowing the patient to breathe ambient air directly without flowing through the nasal interface.

While Figure 3 shows the patient using the invention for mobility, the invention can also be applied to sleep disordered breathing. In the later case, an advantage of the invention is that the mask and tubing is smaller than standard sleep apnea therapy masks and tubing. Additionally, the patient can have the sensation of breathing ambient air more directly making the therapy tolerable to the patient, rather than breathing through a machine, which is the sensation when using standard sleep apnea ventilation devices.

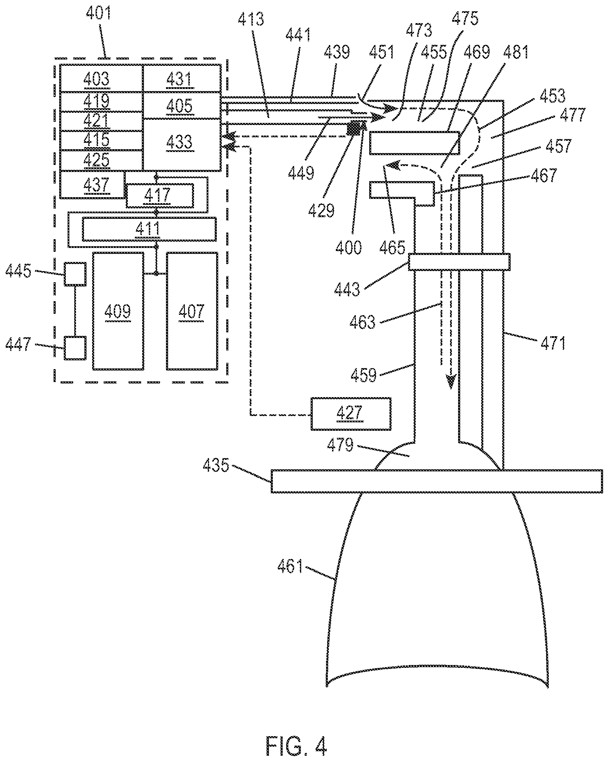

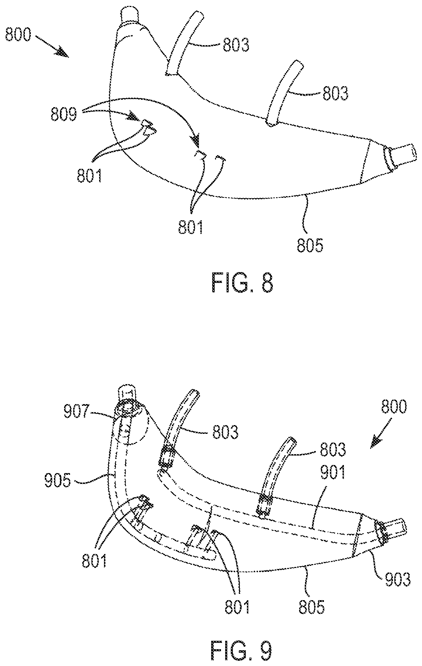

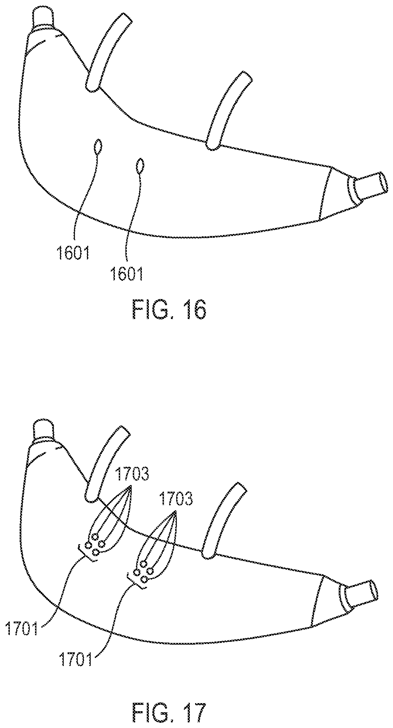

Figure 4 is a block diagram describing an exemplary system of the invention. The exemplary system of Figure 4 may be a wearable ventilator with portable gas source as shown in Figure 3, or may be a different ventilator and/or gas source. Ventilator and patient interface features associated with the system are shown schematically. Figure 4 depicts a non-invasive open nasal interface 400. The non-invasive open nasal interface will be described in various embodiments described herein, for example, in Figs. 5 - 8B (curved nasal mask), Figs. 9 - 15 (flexible joint), and Figs. 16 - 25 and 29 - 31 (ergonomic configuration).

A ventilator module 401 may include or is in communication with several other functional accessories. The ventilator and the patient's internal anatomy from Figure 3 are shown in schematic format in Figure 4. A nasal airway pressure sensor 429 is typically included. A transmitter 403 may be included to transmit information regarding the patient, the patient's therapy, and the ventilator performance to a remote location for review, analysis, remote intervention, two-way communication, and archiving. For example, the patient's compliance with the therapy or utilization of the therapy can be monitored and assessed. Important information can be trended, for example the patient's breath rate, I:E ratio, oxygen usage, activity level, or depth of breathing. Also, information can be sent to a ventilator 433, such as for example, sending programming instructions for setting titration options for the ventilator output to meet the needs of the patient, or sending instructions to the patient. The patient can also send information or questions to a remote clinician through the ventilator and transmitter 403.

An oxygen source 407 and/or a compressed air source 409 can be included, typically external to the ventilator module 401. In certain embodiments, however, the oxygen source 407 and/or the compressed air source 409 can be internal to the ventilator module 401 if the therapy is being used for stationary use, for example, in the home. A blender 411 can be included to control the fractional delivered 02 in a gas delivery circuit 413. A pulse oximeter 415 can be used to titrate settings of the ventilator module 401 to meet the physiological needs of the patient, for example setting the correct oxygen blender setting or ventilator volume output. In addition to compressed supplies of oxygen and air gas, the ventilator module 401 can include internal or external air and oxygen generating systems 417, such as a compressor, pump or blower to create pressurized air, an oxygen generator and/or pump to create pressurized oxygen gas, and/or a compressed gas accumulator. The oxygen source can also be liquid oxygen, or a liquid oxygen generating system. An internal or external humidifier 405 can be included for extended uses of the therapy, or if using in dry climates.

As the therapy is frequently used to help ADL's, and to promote activity, a pedometer 419 and/or actigraphy sensor 421 can be included internal to or external to a ventilator module 401. Optional sensors may include a CO₂ sensor 425, and/or an external breathing sensor unit 437. A CO₂ sensing line 439 and/or an airway pressure sensing line 441 may be present. One or more other external sensors may be included. For example, other external sensors may include an external respiration sensor or respiration effort sensor 427, such as a respiratory muscle effort sensor, a chest impedance sensor 435, or other types of sensors, such as a tracheal or other microphone or vibration sensor 443 or acoustical or ultrasonic sensor. The one or more external sensors may be used either as a redundant sensor to a nasal airflow or nasal pressure sensor 429, or to complement the information obtained from the nasal airflow or nasal pressure sensor 429, or in place of the nasal airflow or nasal pressure sensor 429. An oral airflow breathing sensor may also be used, for example nasal airflow or nasal pressure sensor 429 may alternatively be an oral airflow sensor.

A drug delivery module 431 can be incorporated internally or externally to a ventilator module 401. Because of the challenges with current aerosolized drug delivery inhalers, the drug delivery module 431 can be used to propel and deposit medication particles deep in the respiratory system without a carrier propellant. Because the patient's using the therapy often may also require prescription medication, this may be a convenient and efficient way to administer the medication.

When the therapy is being used for respiratory support, the user may have two options: (1) wearing or toting the ventilator module 401 so that the user can be ambulatory or enjoy the activities of daily living, or (2) stationary use, in the event the patient plans on being stationary or does not have the ability to ambulate. For the later, the delivery circuit can optionally be provided in a 7.62-30.48 m (25-100 foot) length, such that the gas source and ventilator module 401 can be stationary in the patient's home, while the patient can move around their home while wearing the interface and receiving the therapy. Or, the gas source can be stationary, and connected to the ventilator module 401 with a 7.62-30.48 m (25-100 foot) hose, so that the patient can wear or tote the ventilator and be mobile within the range of the hose.

The ventilator module 401 may include one or more processors 445 and one or more memories 447 to analyze information and output therapies.

Ventilation gas 449 may exit at a speed that entrains ambient air 451, such that the combination of ventilation gas 449, entrained ambient air 451 and spontaneously inhaled air, if the patient is spontaneously breathing, is delivered 453 to the patient's airways, such as the nasal cavity 455, oropharyngeal airway 457, trachea 459, lung 461 and others, under power to create a clinically efficacious effect on the lung and airways. Patient may exhale 463 through the nose or mouth. Various airways are also included, such as nostril airway 473, nasal airway 475, oral airway 481, upper airway 477, and lower airway 479.

When using the invention, the patient breathes normally through their upper airway and through their nose, while receiving mechanical support through the interface. During exhalation, the exhaled gas preferably does not enter the gas delivery circuit but rather exits the nose or mouth directly to ambient air, or through, across or around the nasal interface 400 to ambient air. The patient can keep their mouth closed during use for example during inspiration, to help direct the mechanical support to the lower airways and past the oral cavity 465, base of the tongue 467, palate 469 and esophagus 471, or can use a mouth guard or chin band, if necessary. The patient may exhale through their mouth when using the therapy.

Figures 5 - 26 describe embodiments of the non-sealing open-airway nasal mask with nozzles in free space. Systems and methods are described for ventilating a patient in a manner that unencumbers the user, by using a nasal ventilation patient interface and system that allows the user to breathe ambient air around the interface. A gas delivery nozzle may be associated with the nasal interface at a distance from a nose. The nozzle is connectable to a gas delivery circuit and ventilator, and delivers gas front the nasal interface toward the nose. The nasal interface and the nozzle create a negative pressure area near the nozzle, and a positive pressure area near the entrance to the nose. A combination of gas from the ventilator and entrained air are delivered to the patient to support the work of breathing.

Figure 5 describes an embodiment of the invention, showing a ventilation nasal mask assembly 500 with a nasal mask 501 configured to be placed under a nose 503 of a user, without sealing or impeding ambient air from freely flowing in and out of the nose 503. The nasal mask 501 may include a manifold 505. The nasal mask 501 may also include one or more breathing pressure sensing ports 507 or sensors, which are positioned close to an entrance to the nares. The nasal mask 501 may include one or more gas delivery nozzles 509 spaced a distance away from the entrance to the nose 503. The one or more gas delivery nozzles 509 may direct ventilation gas into the nasal airway, and entrain ambient air into the nasal airway.

Gas delivery tubing 511 and pressure sensing tubing 515 from a ventilator, as shown in Figure 4, may be coupled to the manifold 505 at proximal ends of the manifold 505. The gas delivery tubing 511 and pressure sensing tubing 515 may be routed bilaterally away from the manifold 505 and around ears 513 of the user.

Figure 6 shows an isometric view of the nasal mask assembly 500, including the nasal mask 501 at the distal end of the nasal mask assembly 500, gas delivery tubing 511 and pressure sensing tubing 515 attached to the manifold 505 at a distal end of the gas delivery tubing 511 and pressure sensing tubing 515, a Y connector 601 joining the gas delivery tubing 511 and pressure sensing tubing 515 at proximal ends of each arm of the gas delivery tubing 511 and pressure sensing tubing 515, and a combined gas delivery and pressure sensing tubing 603 extending from the Y connector 601 to a ventilator connector 605.

In certain embodiments, a rotatable joint 517 between the gas delivery tubing 511 and manifold 503 and/or a rotatable joint 519 between the pressure sensing tube 515 and manifold 503, may include detent settings. These detent setting joints 517, 519 can be used to adjust the angle of the manifold 503 to adjust the angle of the gas delivery nozzles 507 to be in alignment with the patient's nostril airway. Alternatively, the gas delivery tubing 511 and pressure sensing tubing 515 can be connectable to the manifold 503 in different rotational orientations to likewise align the gas delivery nozzles 507 with the patient's nostril airway.

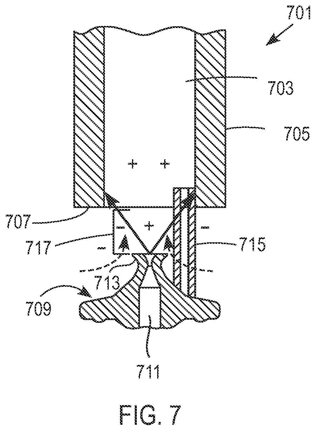

Figure 7 describes a front view cross-sectional schematic representation of a nasal mask 701, showing one exemplary side of the nasal mask 701, for example, the left side. Figure 7 shows a nostril airway 703, a nostril wall 705, a nostril entrance 707, a gas delivery nozzle 713, a gas flow channel 711 through the gas delivery nozzle 713, a manifold 709 that integrates the gas delivery nozzle 713, a breathing pressure sensing cannula 715, and a distance 717 between the gas delivery nozzle 713 and the nostril entrance 707. A rounded distal tip of the gas delivery nozzle 713 may be used to assist in reduction of sound generated by gas exiting the gas delivery nozzle 713.

Figure 7 shows a specific nozzle tip to illustrate that the nozzle tip might be flared or rounded for sound mitigation. The flare is an option.

### Distance from nozzle to nose:

The gas delivery nozzle 713 may be integrated into a manifold 709, and the manifold 709 may be shaped, dimensioned and configured to position the gas delivery nozzle 713 at an ideal position under a nostril entrance 707. A distance of the gas delivery nozzle 713 to the nostril entrance 707 may be chosen to optimize the function of the Venturi created by the gas delivery nozzle 713 and the nares. Optimal function may be described as generating maximal pressure in the nostril airway 703 while the gas delivery is still comfortable and tolerable to the user.

Typically, laminar positive pressure flow should be developed before the airflow reaches deep into the nostril. This positive pressure flow may be defined by the area inside and distal to the gas flow cone defined by the gas exiting the gas delivery nozzle 713. The area outside of this cone is negative pressure created by the Venturi, which entrains ambient air into the nose and nasal passage, thus generating the energy required for mechanical ventilatory support. When this cone intersects with the internal wall of the nostril, the distal side of that intersecting point is positive pressure.

Alternatively, based on position of the gas delivery nozzle 713 and other operational parameters and device dimensions, this cone can be wider than the entrance to the nostril when it reaches the nostril. In this event, positive pressure occurs outside of the nostril and extends distally. Also alternatively, this cone can intersect with the nostril walls at a distance inside the nostril, thereby allowing a negative pressure zone to occur at the entrance to and slightly inside the nostril, but then transitioning to positive pressure distal to the intersecting point. Because the cross sectional geometry is non-uniform, for example, not a perfectly circular, there is variability with the gas flow cone intersecting points with the nostril wall, around the circumference of the cone and nostril. As will be described subsequently, specific embodiments of the nasal mask may address this nuance such that more uniform and predictable performance can be achieved.

In the embodiment of Figure 7, the nostril entrance 707 may act as a jet pump inlet and the nasal passage may act as a jet pump throat. Intuitively, it would be expected that optimal function would dictate placing the gas delivery nozzle 713 at the nostril entrance 707 or slightly inside the nares; however, it was determined through empirical testing than in the average adult user, the optimal position for the gas delivery nozzle 713 to achieve optimal Venturi function is to place the gas delivery nozzle 713 approximately 0.950 inches from the nostril entrance 707.

### Position of breathing pressure sensing port:

For embodiments of the invention to be effective, it may be necessary to measure and monitor breathing of the patient to properly synchronize a ventilator gas delivery control system with spontaneous breathing patterns of the patient, as desired clinically. Therefore, while the gas delivery nozzles 713 may be positioned ideally at a distance away from the user's nostril entrance 707, breathing pressure sensing cannula 715, breathing pressure sensing ports or other sensors may need to be placed near, at or inside the nostril entrance 707. For example, the distal end of the pressure sensing cannula 715 can be placed slightly inside the nose in the area where positive pressure has been created by the Venturi system.

It may be beneficial to have multiple locations for measuring pressure. For example, one location may be used for detecting and measuring the spontaneous breathing pressure of the patient, and a different location for measuring the pressure generated by the ventilation system. For example, a breathing pressure sensing port may be placed slightly inside the nostril entrance 707, and a ventilation gas pressure sensor may be placed outside the nostril entrance 707, or alternatively deeper inside the nostril airway 703.

The location of pressure sensing ports, such as the breathing pressure sensing cannula 715, may be selected to optimize accuracy and fidelity. For example, a breathing pressure sensing port, such as the breathing pressure sensing cannula 715, may be arranged so that it is located near the medial aspect of the nostril airway 703, or at the posterior aspect of the nostril airway 703. Multiple breathing pressure sensing locations may also be used. For example, a sensing port at a medial posterior aspect of the nostril airway 703 may be used to measure inhalation pressures accurately, and a sensing port at the anterior aspect of the nostril airway 703 may be used to measure exhalation pressures accurately.

In addition to a nostril airway breathing pressure sensor, other sensor types or locations may be used. For example, a microphone or ultrasonic sensor can be used to detect phases of breathing when placed on the user's neck to detect movements of air in the trachea. Other sensors and sensor locations can be used.

In addition to the ventilation pressure being measured by a pressure sensing port outside of the nose, at the nostril entrance, or inside the nostril airway, the ventilation pressure can be derived by other apparatus and methods. For example, a gas delivery pressure in the gas delivery circuit can be correlated to a delivered ventilation pressure that is delivered to the patient by the ventilation system by measuring key relevant patient parameters, such as airway resistance and respiratory track compliance, and correlating those parameters with delivered pressure based on a gas delivery pressure.

Figure 8 shows a top-side view of the nasal mask 800 of an alternative embodiment of Figure 5, showing gas delivery nozzles 809 and pressure sensing cannula 803. The gas delivery nozzles 809 may include a pair of exit ports 801 for both the right and left gas delivery nozzles 809. As discussed later, the dual exit ports 801 may improve the function and user tolerability of the device.

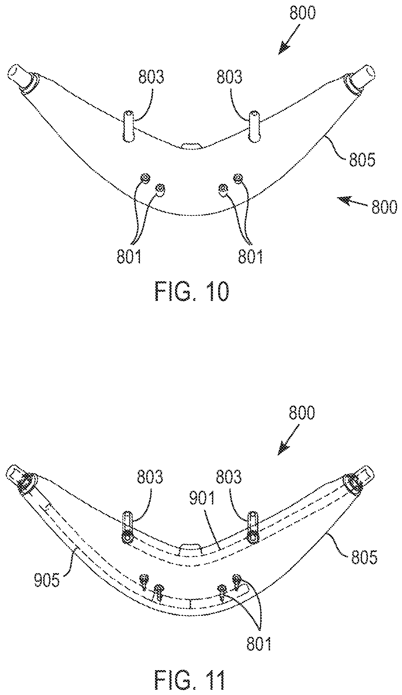

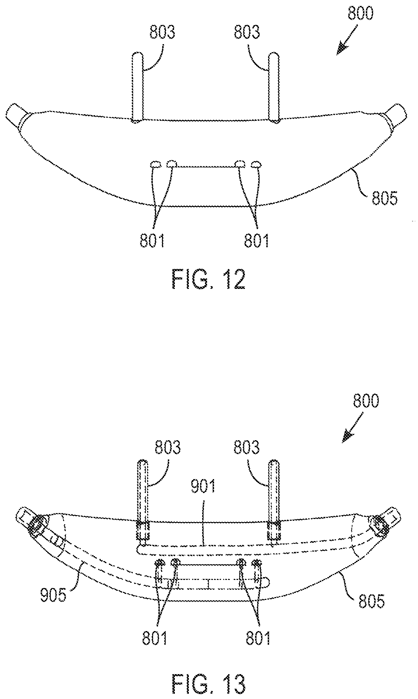

Figure 9 shows a hidden line view of the nasal mask 800 and manifold 805 shown in Figure 8, including a pressure sensing lumen 901 running from a first proximal end 903 of the manifold 805 to and through each of the pressure sensing cannula 803, and a gas delivery lumen 905 running from a second proximal end 907 of the manifold 805 to and through each of the exit ports 801. Figure 10 shows a top view of the nasal mask 800 shown in Figure 8, and Figure 11 shows a hidden line view of the nasal mask 800 shown in Figure 10. Figure 12 shows a front-top view of the nasal mask 800 shown in Figure 8, and Figure 13 shows a hidden line view of the nasal mask 800 shown in Figure 12.

Key dimensions and values of the ventilation nasal mask are indicated in Table 1. The parameters provided by the ventilation nasal mask and system are indicated in Table 2. Additional exemplary dimensions, values and materials of the ventilation nasal mask are indicated in Table 3.

### Nozzle patterns:

In certain situations, delivery of ventilation gas to the patient through one left and one right gas delivery nozzles may not develop the laminar flow desired due to the variability found in patient's nostril and nasal air passage geometries. Therefore, in certain embodiments of the invention, the mask's left and right gas delivery may each be performed by multiple nozzles.

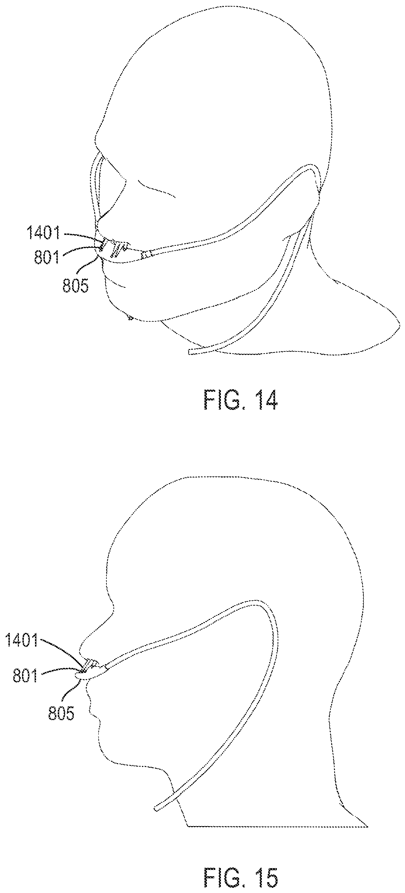

For example, as shown in Figure 8, a pair of left and a pair of right gas exit ports 801 may be incorporated in the manifold 805, such that the pattern created by the gas exiting the exit ports 801 is spread out in a pattern approximating the cross-sectional area of the nostril airway, and thus facilitating creating laminar positive pressure flow. The pattern 1401 created by flow emission from the exit ports 801 is shown in Figures 14 and 15. This flow and pressure head profile spreads out and smoothes out the flow profile, which may optimize the flow characteristics, and facilitate creating laminar positive pressure flow with minimal disturbance and resistance. Other embodiments may also be used. For example, an oval shaped gas delivery nozzle orifice 1601, as shown in Figure 16, or a gas delivery nozzle array 1701 of individual gas delivery nozzles 1703, for example arranged in a circular or oval pattern, as shown in Figure 17. Any nozzle pattern may be used.

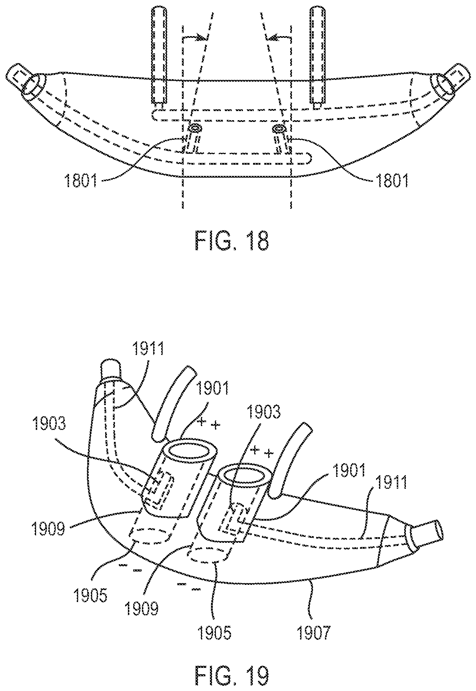

In addition to the gas delivery nozzle pattern, the included angle between the gas flow path axis created by the left and right nozzles or nozzle patterns may be non-parallel. For example, as shown in Figure 18, a nozzle gas flow path 1801 and exit axis can be angled inward, for example at an angle of approximately 0.5-20 degrees inward, and preferably approximately 2-6 degrees inward. This angle may align the ventilation gas flow entering the nostril airway with the nostril airway, which may optimize flow characteristics, and facilitate creating laminar positive pressure flow with minimal disturbance and resistance.

Figures 14 and 15 describe the gas flow path pattern entering the nose. As an example, a dual left and dual right gas delivery nozzle pattern is used in this description. As can be seen, the combined gas flow pattern created in the nostril airway by the dual nozzle arrangement may distribute the delivered flow and velocity profile evenly across the proper cross-section of the nostril airway path. This may improve positive pressure formation before the gas travels deep into the nasal airway, improve laminar flow, and reduce turbulence that could be irritating to the user. For example, if the flow profile is more concentrated, a high velocity flow can impinge on a nerve receptor on the inside of the nasal passage, which could be intolerable to the user. In testing, more focused flow profiles were found to irritate users, whereas a more distributed flow profile was found to be tolerable. The spacing, angle, rotational position, and/or orientation of the nozzles can be adjustable, or the mask can be available in different sizes, so that the flow pattern created by the nozzles matches the size and shape of the user's nasal anatomy. For example, when dual nozzles are used, the nozzle positions can be rotated to rotate the rotational position of the oval pattern created by the pair of nozzles, so that the oval pattern matches with the oval orientation of the user's nostril airway.

### Nasal mask with jet pump throat:

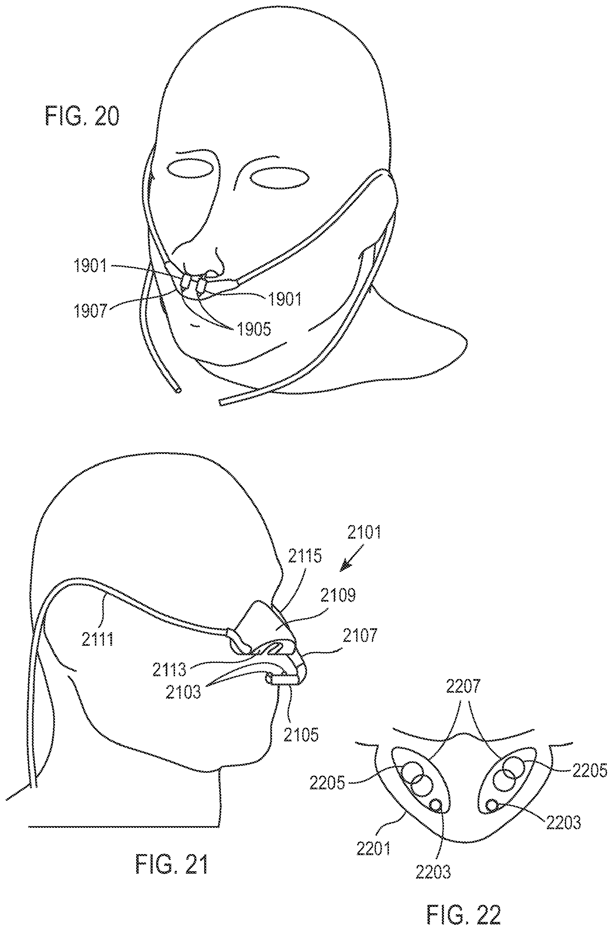

In addition, as shown in Figures 19 and 20, the mask can include a jet pump throat section 1901. Figure 19 has an entrainment port 1905 opening on bottom of a manifold 1907. Figure 20 shows same throat section 1901, but has the entrainment ports 1905 on top of the manifold at the base of the throat section 1901. In Figure 19, ambient air may be entrained through the bottom of the manifold 1907. In Figure 20, ambient air may be entrained past the top of the manifold 1907.

The jet pump throat section 1901 can be useful in creating consistent performance of the ventilation system from one person to another, by minimizing the effect of patient anatomy on performance. The jet pump throat section 1901 can also be useful in dampening the sound that is generated by the high velocity gas exiting gas delivery nozzles 1903 and entraining ambient air. The jet pump throat section 1901 can alternatively include entrainment ports 1905 at the base of the jet pump throat section 1901 as shown in Figure 20, or a manifold 1907 can include a through-hole 1909 that functions as an entrainment aperture as shown in Figure 19, which extends through the thickness of the manifold 1907 from the bottom of the manifold 1907 to the top of the manifold 1907 and which is in communication with the gas delivery nozzle 1903. The throat section 1901 of the mask shown in Figure 19 can also be used to reduce the sound generated by the Venturi and as such this embodiment may be useful in a sleep apnea application, in which minimal sound is a critical performance requirement. The entrainment port 1905 is shown at bottom of manifold 1907 in Figure 19, but can be on the side of the jet pump throat section 1901, for example, near the nozzle 1903. Gas delivery lumens 1911 may be included from either proximal end of the manifold 1907.

The nozzle in Figure 19 can be any type of nozzle. In Figure 19, there may be a throat option that is part of the manifold and outside of the nose.

### Other mask form factors:

Figure 21 describes an alternative embodiment of a nasal mask 2101 in which gas delivery nozzles 2103 are positioned in the correct location under the nose by use of a horizontal extension arm 2105 attached to a vertical extension arm 2107 that extends down from a nose piece 2109 that is configured to be placed on and secured to the front of the nose. The nose piece 2109 can be secured to the nose by a variety of means, such as by using gas delivery tube 2115 and pressure sensing tube 2111 connected to the nasal mask 2101 to secure the nose piece 2109 to the face. The nose piece 2109 can also be secured to the nose by other means, such as by straps, adhesives, a friction fit, or combinations thereof.

The vertical extension arm 2107 can be adjustable to position the gas delivery nozzles 2103 at the appropriate distance from the user, and the horizontal extension arm 2105 can be rotate-ably adjustable to angle the gas delivery nozzles 2103 correctly to be in alignment with the nostril airway. The spacing between the gas delivery nozzles 2103 can be adjustable, for example by a linear adjustment in the horizontal arm.

Breathing pressure sensing ports (not shown) may extend upward from the nose piece 2109 to be positively located at, near or inside the entrance to the nose. The nose piece 2109 may include a shelf 2113 at its bottom end which is used to position against the outside of the nostril rim. The breathing pressure sensing tube 2111 may be attached to one side of the nose piece 2109, the user's right side in Figure 21, and the gas delivery tube 2115 may be attached to the opposite side.

The nasal mask 2101 may also include additional sensing functions such as a CO2 gas sampling port (not shown) and conduit extending to a capnometer (not shown), which can be included by integrating a secondary channel into the gas delivery tubing or pressure sensing tubing, and integrating the requsite channel into the mask nose piece and or extension arms. The nose piece 2109 may also prevent gas being delivered from the gas delivery nozzles 2103 from being directed toward the eyes when the nasal mask 2101 is not fitted properly to the user.

The nasal mask 2101 may also include additional sensing functions such as a CO₂ gas sampling port (not shown) and conduit extending to a capnometer (not shown). The nose piece 2109 may also prevent gas being delivered from the gas delivery nozzles 2103 from being directed toward the eyes when the nasal mask 2101 is not fitted properly to the user.

This embodiment of the invention may use the angle of the medial aspect of the bridge of the nose to align the therapy to the patient. During testing, it was determined that the optimal performance was achieved when the gas delivery nozzles 2103 were aimed parallel to the bridge of the nose to align the jets of ventilation gas with the nares. The gas delivery nozzles 2103 of the nasal mask 2101 may be aimed parallel to the nose piece 2109, such that by placing the nose piece 2109 on the bridge of the nose, the gas delivery nozzles 2103 may be parallel to the bridge of the nose.

If there is some misalignment, performance may degrade. The gas delivery nozzles 2103 preferably are kept within 10 degrees of being properly aligned with a nasal opening and an axis of the nares. As such, when a patient moves their nose to the left or right (e.g. by moving your jaw in an exaggerated manner), the nasal mask 2101 may follow the nose, ensuring that the gas delivery nozzles 2103 remain aligned with the centerline of the nose, and therefore the nostrils. In Figure 22, gas delivery patterns 2205 may include two intersecting circles to create an effective oval pattern, which are for example generated by dual nozzles, as explained previously for the purpose of developing a laminar cross section of flow and positive pressure in the nostril airway.

Figure 22 shows a bottom view of a nose 2201 and how the nasal mask 2101 of Figure 21 is aligned with the nose 2201. A breathing pressure sensing location 2203 and gas delivery patterns 2205 are superimposed on the bottom view image of a nose 2201 and the nostril airway 2207 as depicted by the two large oval shapes. Gas delivery pattern 2205 and nasal air pressure sensing locations 2203 are indicated by the large and small circles, respectively. The patterns 2205 are generated in this example by two gas delivery nozzles for the left and right nostril, which applies to other mask embodiments of the invention in addition to the mask of Figure 22.

The nasal air pressure sensing ports may be protrusions to help achieve a positive location of the sensing ports in the breath path in the nares. The gas delivery ports may be positioned such that the gas delivery path has a clear path to the nostril airway. There may be two or more sizes of nasal mask 2201, and or adjustment features in the mask, so that the sensing ports and gas delivery zones are properly aligned with the nasal airway path. The previous figures describe that the sensing locations must be in proximity to the entrance of the nostril, either inside, coplanar to the entrance, or slightly outside but if outside no more than 5mm away from the entrance, whereas the gas delivery nozzle tips are located a distance from the entrance to the nostrils, for example 10-25mm away. This configuration may allow the nasal mask 2201 to take advantage of the jet pump geometry, while not sacrificing sensing accuracy, so that the ventilator is in proper synchrony with the patient. Also, the gas flow profile may become more organized before entering the patient's nostril, rather than a turbulent jet entering the nostril, which would be quite uncomfortable and intolerant to the patient.

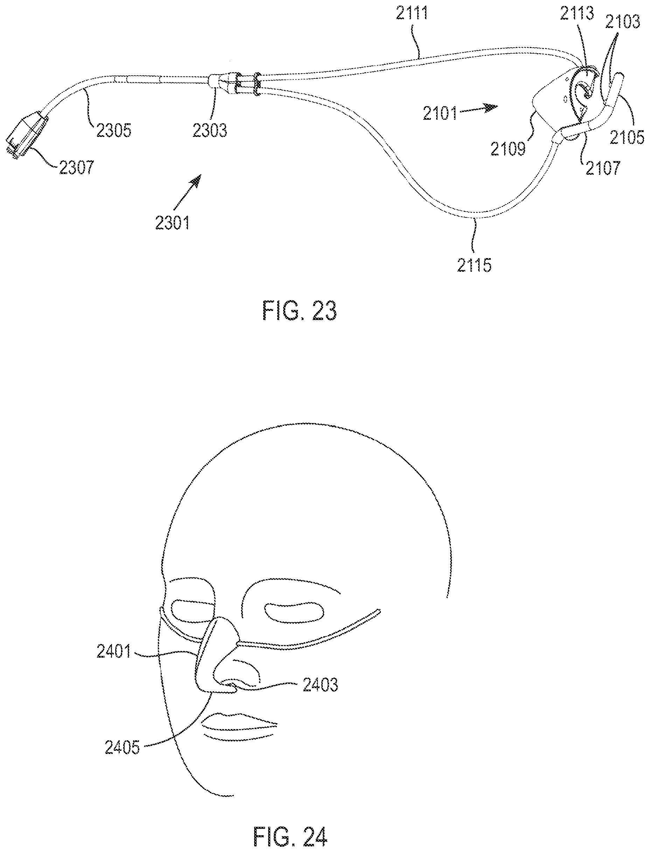

Figure 23 shows an isometric view of the patient circuit assembly 2301 of the nasal mask 2201 shown in Figure 21. A Y connector 2303 joining a breathing pressure sensing tube 2111 and gas delivery tube 2115 is shown, and combined tubing 2305 and a ventilator connector 2307 at a proximal end of the patient circuit are shown.

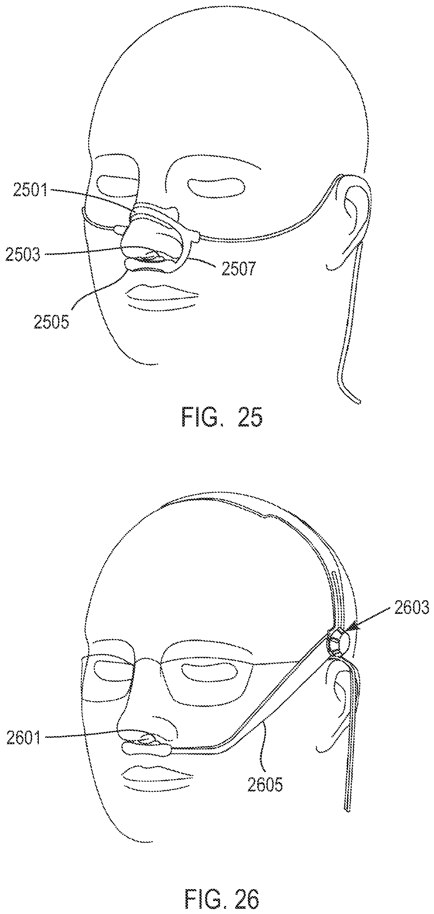

Figures 24 and 25 describe aesthetically streamlined versions of the nasal mask shown in Figure 21. Features of the nose pieces 2401, 2501 may be trimmed to optimize the comfort and to minimize the obtrusiveness to the user. In Figure 24, the gas delivery nozzles 2403 may be positioned under the nose by an extension 2405 of the nose piece 2401 itself versus the vertical extension arm of the mask in Figure 21. In Figure 25, the nose piece 2501 may be a strip on the top of the bridge of the nose and the gas delivery nozzles 2503 may be positioned by use of a streamlined vertical arm 2505 and a streamlined horizontal arm 2507.

Figure 26 shows a version of the mask shown in Figure 21 in which gas delivery nozzles 2601 are positioned under the nose at the correct location using head gear 2603 with an extension arm 2605. The head gear 2603 and extension arm 2605 can be adjustable to position the gas delivery nozzles 2601 and breathing pressure sensing ports (not shown) in the correct location.

The following tables list exemplary values only and are not to be construed as limiting the disclosure.

**Table 1: Nasal Mask Exemplary Key Dimensions and Values**

| **Feature** | | |
|---|---|---|
| | Preferred/ideal | Range |
| Nozzle diameter: | 0.033 in | .010-.050 in |
| Flow rate delivered to nozzle: | 30 lpm | 6-40 lpm |
| Input pressure delivered to nozzle: | 35 psi | 5-60 psi |
| Throat length, if included: | .6-1.0 in | .3 - 1.5 in |
| Throat typical cross sectional area, if included: | 0.04 in² | 0.02-0.06 in² |
| Nozzle distance to proximal edge of nose: | 0.5-1.2 in | 0.3-1.3 in |

**Table 2: Exemplary Ventilatory Support Parameters**

| **Parameter** | **Range** | Preferred (Adult*) |
|---|---|---|
| Lung Volume Augmentation (%) | 10-150% | 15-65% |
| WOB reduction (%) | 5-80% | 10-50% |
| Lung Pressure increase (cmH₂O) | 1-30 | 3-20 |
| Upper Airway pressure increase (cmH₂O) | 3-34 | 7-25 |
| Lung Pressure or Volume Waveform | (1) | R |
| Entrained ambient air (% of Ventilator gas delivery) | 10-300% | 50-100% |
| Gas exit speed out of gas delivery nozzle (m/sec) | 25-350 | 50-200 |
| Ventilator Output flow rate, average (lpm) | 5-40 | 10-20 |
| Gas Delivery Tubing outer diameter (mm) | 3-7 | 4-6 |
| Ventilator Output Pressure (psi) | 10-60 | 20-40 |
| Ventilator Drive Pressure (psi) | 10-80 | 20-50 |
| Ventilator Operating Pressure (psi) | 5-40 | 25-35 |
| Ventilator Output Volume (ml) | 10-750 | 50-350 |
| Ventilator Output Pulse Time (sec.) | 0.100-1.25 | 0.200-0.750 |
| Therapy's nominal source gas consumption (lpm) | 0.5-6.0 | 2-4 |
| Ventilator Output Synchronization (ms) | variable depending on comfort and need (25-500ms delay) | variable depending on comfort and need (75-250ms delay) |
| Ventilator Output Waveform | (1) | Descending |

**Table 3: Additional Exemplary Dimensions, Values and Materials**

| **Feature** | | **Range** | **Preferred Range** |
|---|---|---|---|
| **Dimensions** | | | |
| Gas delivery hose, ID (mm) | | 2.0-7.0 | 2.5-4.5 |
| Gas delivery hose, Length (ft), ambulating with wearable system | | 2-6 | 2.5-4 |
| Gas delivery hose, Length (ft), ambulating with stationary system | | 20-75 | 40-60 |
| Gas delivery hose, Length (ft), sleeping | | 4-15 | 6-10 |
| Jet Nozzle, Length (mm) | | 0.5-15 | 2-10 |
| Manifold Length (mm) | | 20-160mm | 30-80mm |
| Manifold spontaneous breathing gas flow path volume | | 0 ml | 0 ml |
| Manifold pressure sensing line diameter (in) | | .008-.055 | .015-.040 |
| Manifold breathing resistance (cmH₂O @ 60 lpm) | | 0 | 0.01-0.10 |
| Breathing sensing port, distance to nose (mm) | | -5 to 2 | -10 to 5 |

| **Materials** | **Types** | | **Preferred** |
|---|---|---|---|
| Gas delivery hose | PP, PE, PS, PVC | | PE |
| Cannula | PU, PVC, Silicone | | PVC, Silicone |
| Manifold | PVC, Silicone, PU, PE, Polysolfone | | PVC, Silicone |
| Jet Nozzle | Metal, Ultem, Nylon, LCP, PVC, PC, ABS, PEEK | | PVC |

| | | | |
|---|---|---|---|
| (1) Square, Rounded, Descending, Ascending, Sinusoidal, Oscillating. * Dimensions listed are exemplary and for average sized adults; pediatric sizes 20% less, neonatal sizes 50% less. | | | |

Diameters listed are effective diameters (average cross sectional dimension).

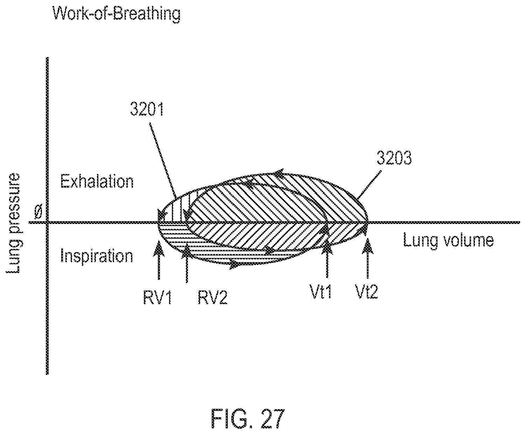

Figure 27 describes the mechanism of action of the invention, and how the patient's work of breathing may be beneficially affected by the invention, when the invention is used for lung disease or neuromuscular disease applications. The patient's lung volume may be graphed as a function of lung pressure, the area inside the curve representing work, typically expressed in Joules per Liter (J/L), and for a normal healthy adult can be 0.3-0.6 J/L. For a respiratory compromised patient, 4-10 times more work can be required to breathe during rest, and even more during exertion, to overcome the diseased state of the tissue, for example to overcome static and dynamic hyperinflation as in the case of COPD, or to overcome high airways resistance as in the case of fibrosis or ARDS.

In the graph shown, the area inside the curve below the pressure axis is the inspiratory WOB, and the area defined by the area inside the curve above the pressure axis is the expiratory WOB. The arrows show the progression of a single breath over time, starting from RV to VT then returning from VT to RV. RV1 and VT1 are the residual volume and tidal volume without the therapy. Line 3201 represents spontaneous breathing without non-invasive open nasal ventilation. Line 3203 represents spontaneous breathing with non-invasive open nasal ventilation, with inspiratory augmentation and positive end-expiratory pressure (PEEP) therapy. RV2 and VT2 are the residual volume and tidal volume with the therapy. As can be seen, RV increases with the therapy because in this example, expiratory flow is provided as part of the therapy, which may increase residual volume. Importantly, VT is increased with the therapy and is increased more that the RV is increased, indicating that more volume is entering and leaving the lung as a result of the therapy. The increase in tidal volume is considered clinically efficacious, however is technically challenging to achieve in an open ventilation, non-invasive and minimally obtrusive system. As is shown in the graph, the patient's inspiratory WOB with the invention ON may be about 25% less than the patient's inspiratory WOB with the invention OFF. Also, inspiratory lung pressure increases (is less negative) and tidal volume increases, and optionally exhaled pressure increases if the therapy is provided during exhalation. While residual volume increases in the example shown because the ventilator is providing gas in this example during the expiratory phase, the ventilation parameters can be titrated to not effect residual volume, and because of the ability of the patient to exercise their lung muscles when receiving the therapy, the patient's lung mechanics may remodel in the case of COPD, actually causing a reduction of residual volume to a more normal value. In the graph shown, the waveform with therapy assumes an early inspiratory trigger time for the ventilator inspiratory phase therapy output, and that the volume output is delivered within the patient's inspiratory time. Optionally, however, different delivery waveforms and delivery synchronizations can be performed, which may adjust the WOB curve. For example, the ventilator inspiratory phase therapy can be delivered late in the person's inspiratory cycle, with delivery completing at the end of inspiration, and delivered with a square or ascending waveform profile. In this case the WOB curve with therapy will be tilted upward to the right of the curve, such that inspiration ends and transitions to exhalation at a point above the lung pressure zero axis.

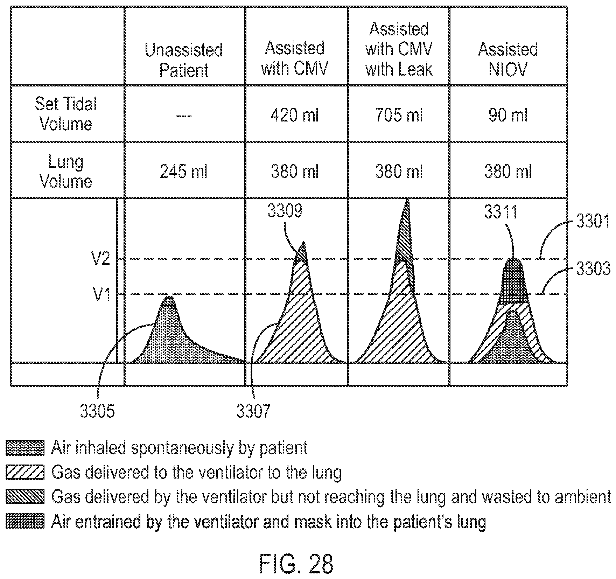

Figure 28 graphically illustrates the lung volumes achieved with NIOV on a lung simulator bench model in comparison to conventional ventilation. In all the waveforms the simulated patient is spontaneously breathing at the same inspiratory effort which results in a tidal volume of 245 ml, and the clinical goal is to increase the patient's tidal volume from 245ml 3301 to 380ml 3303. In the first waveform from left to right in the graph, the patient's breath 3305 is un-assisted and thus the patient receives a tidal volume of 245ml. In the next waveform, the simulated patient with the same effort is assisted with a traditional closed system ventilator, such as with a sealed breathing mask or cuffed airway tube. The ventilator output 3309 is set to a level to achieve the desired "assisted" tidal volume of 380ml. The ventilator is set to 420ml to achieve this goal, as there is a discrepancy between the gas delivered to the lung by the ventilator versus the gas delivered by the ventilator but not reaching the lung and wasting to ambient 3307. In the third waveform, a small leak is introduced in the conventional ventilator system, such as would be done in the case of weaning the patient off of the ventilator. To achieve the desired "assisted" tidal volume of 380ml, the ventilator must now be set at 705ml. In the second and third waveforms, it can also be seen that all of the volume received by the patient's lung originates from the ventilator, which it must in these conventional systems. In the forth waveform, the patient is assisted with the NIOV, and as can be seen, the NIOV ventilator output only has to be set at 90ml to achieve desired "assisted" level of 380ml. In this case, only some of the 380ml tidal volume comes from the ventilator, and a substantial portion of the 380ml comes from entrainment and spontaneously inspired ambient air 3311, therefore making the NIOV system far more efficient, comfortable, and healthier, than the other systems.

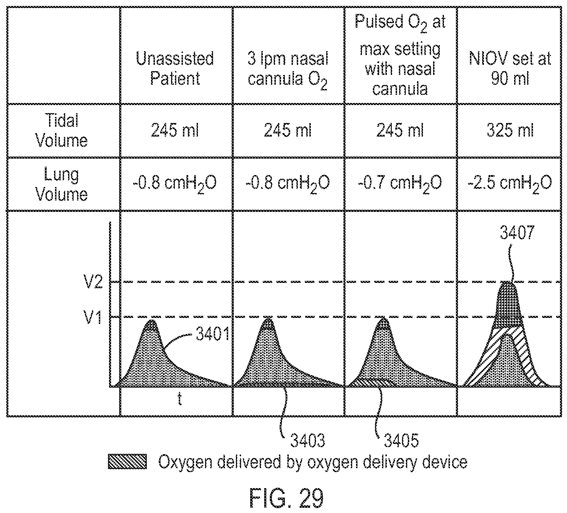

Figure 29 graphically shows NIOV in comparison to oxygen therapy, using the lung simulator bench model. In the first waveform on the left, the patient is unassisted and breathes at an effort of -0.8cmH2O, generating 248ml of inspired tidal volume 3401. In the second waveform and third waveform, the patient receives continuous flow 3403 and pulsed flow 3405 of oxygen respectively via nasal cannula, with no or negligible effect on lung pressure and tidal volume. In the forth waveform, NIOV 3407 is used which shows a marked increase in lung pressure and tidal volume, thus indicating that NIOV helps in the work-of-breathing as described earlier, despite the fact that NIOV is an open airway system.

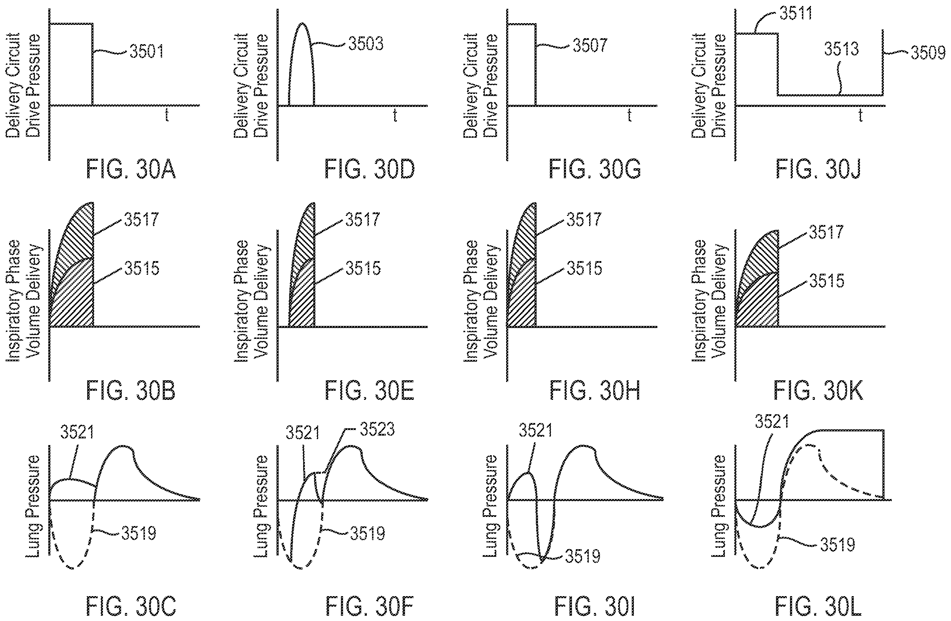

Figures 30A - 30L show exemplary ventilation gas delivery profiles of the invention and their respective effect on lung volume and lung pressure.

Figures 30A, 30D, 30G and 30J show exemplary pressure and/or flow waveforms delivered by the ventilator. Figure 30A describes a square waveform 3501 delivered during the complete inspiratory cycle; Figure 30D describes an ascending and descending waveform 3503; Figure 30G describes a square waveform 3507 delivered for the first part of the patient's spontaneous inspiratory time; Figure 30J shows a multilevel amplitude waveform 3509 with a first amplitude 3511 delivered during the inspiratory phase and a second amplitude 3513 during the expiratory phase, where the second amplitude 3513 for example is used to deliver positive end-expiratory pressure (PEEP), which in some clinical applications will be efficacious. Other waveforms are also included in the invention, such as a descending trapezoidal or ascending trapezoidal square wave. The pressure and flow rate output from the ventilator into the gas delivery tubing is typically in the 5-40 psi and 6-30 lpm range.

Figures 30B, 30E, 30H and 30K describe the lung volume being delivered by the therapy including a ventilator output 3515 and an entrained volume 3517.

Figures 30C, 30F, 30I and 30L show the lung pressure without therapy represented by the dashed line 3519, and the resultant lung pressures with the therapy represented by the solid line 3521, showing a positive inspiratory pressure in Figure 30C for the entire inspiratory phase, a positive inspiratory pressure for part of the inspiratory phase in Figures 30F and 30I, with therapy extending into exhalation 3523, and an elevated negative inspiratory pressure in Figure 30L.

Figures 36A - 36L describe additional exemplary ventilation gas delivery profiles of the invention and their respective effect on lung volume and lung pressure.

Figure 31A describes an ascending waveform 3601. Figure 31D describes a descending waveform 3603. Figure 31G describes a multi-level waveform 3605 with a lower amplitude in the first portion of the inspiratory phase, for example to deliver the necessary oxygen molecules to the lung early in the breath phase, and a higher amplitude in the second portion of the inspiratory phase, for example to deliver the mechanical support portion of the therapy to help the work of breathing. Figure 31J describes an oscillatory waveform 3607, which may be use the gas supply more efficiently while producing nearly the same Venturi, entrainment and therapeutic effect.

Figures 31B, 31E, 31H and 31K describe the lung volume being delivered by the therapy including a ventilator output 3609 and an entrained volume 3611.

Figures 31C, 31F, 311 and 31L show the lung pressure without therapy represented by the dashed line 3613, and the resultant lung pressures with the therapy represented by the solid line 3615.

The lung pressure resulting from the therapy may be governed by a combination of factors: the gas delivery circuit pressure, the jet pump design and configuration, the patient's lung compliance and airway resistance, the patient's breathing effort, the timing of the ventilator output relative to the patient's inspiratory phase, and the ventilator output waveform. Typically, however, a gas delivery circuit pressure of 2.069 bar (30 psi) delivering 100ml with a square waveform, and delivered for 500msec starting at the beginning of the patient's inspiratory phase, may increase lung pressure by 5-15cmH2O. And, typically a gas delivery circuit pressure of 2.069 bar (30 psi) delivering 250ml with a trapezoidal waveform, and delivered for 700msec during the majority of the patient's inspiratory phase, may increase lung pressure by 10-25cmH2O. The gas delivered by the ventilator can be oxygen, air, oxygen-air mixtures, or therapeutic gases such as helium. In a main mechanism of action of the invention, the patient's lung pressure and lung volume is increased, which allows the patient to exert them self without being limited by fatigue and dyspnea. In another main mechanism of action of the invention, the patient reduces their breathing effort in response to the pressure and volume support provided by the therapy, thus resulting in no change in total lung volume from the therapy, but resulting in a reduced work of breathing. In another main embodiment of the invention, a combination of the above two mechanisms of action can occur.

Figure 32 is a diagram of timing and gas flow delivery, according to one embodiment. Amplitude of gas flow delivery rate 3701 modulates with respiratory rate to affect airway pressure 3703. The faster the respiratory rate, the higher the amplitude. The volume delivery may be maintained at a constant rate, unless changed by the user, between the restful state and exertion state. However, the amount of power delivered by the system may be higher during the exertion state, because the faster flow rate entrains more gas, which produces more power and higher lung pressures during inspiratory phase. Further, the delivery time of the delivered flow can be adjusted by the user as a percentage of the breath period. For example, if the breath period is 3 seconds, a 25% delivery time setting would equal a delivered flow pulse width of 0.75 seconds. The delivered flow pulse width would change with the breath rate; however, it may continue to be 25% of the breath period (unless changed by the user). The setting can be set for example in the range of 15% to 70% of the breath period. The setting may be independent of the volume setting. For example, a setting of 25% versus 40% may still deliver the same set volume, and may merely deliver the set volume at different flow rates. The algorithm for adjusting the delivered flow pulse time may, for example, look at the preceding 3 to 5 breaths to determine what the current breath period is, and may have a correction factor to rule out outlier breaths.

Although the foregoing description is directed to the preferred embodiments of the invention, it is noted that other variations and modifications will be apparent to those skilled in the art, and may be made without departing from the scope of the invention as defined by the appended claims. Moreover, features described in connection with one embodiment of the invention may be used in conjunction with other embodiments, even if not explicitly stated above.

## Claims

1. A system for supplying ventilatory support to a patient, the system comprising a gas delivery source (407, 409), a gas delivery circuit (413), and a non-sealing nasal ventilation interface (400), the non-sealing nasal ventilation interface (400) being configured to communicate with a patient's nostril airway while allowing the patient to breathe ambient air directly without flowing through the non-sealing nasal ventilation interface (400), wherein:
the non-sealing nasal ventilation interface (400) comprises a manifold (505), the manifold (505) comprising a nozzle (509) connectable to gas delivery tubing and the gas delivery source (407, 409) and configured for communication with a patient's nostril airway, the manifold (505) being configured to position the nozzle (509) at a distance from a nostril entrance of the patient's nostril airway such that a gas flow cone defined by gas exiting the nozzle (509) intersects with an internal wall of the patient's nostril airway at an intersecting point, the area inside the gas flow cone and the area within the patient's nostril airway distal to the intersecting point defining a positive pressure area wherein a laminar positive pressure flow is developed, the area outside the gas flow cone defining a negative pressure area entraining ambient air into the patient's nostril airway, the combination of gas from the gas delivery source (407, 409) and the air entrained into the patient's nostril airway providing ventilatory support.

2. The system of claim 1, wherein the manifold (505) is configured to position the nozzle (509) at an angle relative to a centerline of a nostril airway.

3. The system of claim 1, further comprising one or more sensors, wherein the one or more sensors comprise a sensing channel that extends distally away from the nozzle.

4. The system of claim 3, wherein the sensing channel is configured to extend into a patient's nostril airway during use.

5. The system of claim 1, wherein the nozzle is configured to create an oval shaped gas delivery flow profile within a patient's nostril airway.

6. The system of claim 1, further comprising a jet pump throat comprising a flow path.

7. The system of claim 1, wherein the system is configured to provide a positive pressure of approximately 0.7 - 34.5 mBar (0.01 - 0.50 psi).

8. The system of claim 1, wherein the system is configured to allow the nozzle to be positioned up to 3.8 cm (1.5 inch) outside the entrance to the patient's nostril airway.

9. The system of claim 1, wherein the system is configured to allow the delivery of gas through the nozzle to be synchronized with a breathing pattern of the patient.

10. The system of claim 1, further comprising a wearable ventilator (401), wherein the gas from the gas delivery source (407, 409) is controlled by the wearable ventilator (401).

11. The system of claim 1, wherein the provided ventilatory support mechanically contributes to reducing the work of breathing to treat respiratory insufficiency.

## Patentansprüche

1. System zur Bereitstellung von Atemhilfe für einen Patienten, wobei das System eine Gaszufuhrquelle (407, 409), einen Gaszufuhrkreis (413) und eine nicht-abdichtende nasale Beatmungsschnittstelle (400) umfasst, wobei die nicht-abdichtende nasale Beatmungsschnittstelle (400) zur Kommunikation mit dem Atemweg über das Nasenloch eines Patienten konfiguriert ist, während dem Patienten erlaubt ist, direkt Umgebungsluft zu atmen, ohne dass sie durch die nicht-abdichtende nasale Beatmungsschnittstelle (400) zu strömen braucht, wobei:
die nicht-abdichtende nasale Beatmungsschnittstelle (400) ein Verteilerstück (505) umfasst, wobei das Verteilerstück (505) eine mit dem Gaszufuhrschlauch und der Gaszufuhrquelle (407, 409) verbindbare Düse (509) umfasst und zur Kommunikation mit dem Atemweg über das Nasenloch eines Patienten konfiguriert ist, wobei das Verteilerstück (505) zur Positionierung der Düse (509) in einem Abstand vom Nasenlocheingang des Atemwegs über das Nasenloch des Patienten derart, dass ein Gasflow-Konus, definiert durch die Düse (509) austretendes Gas, mit einer Innenwand des Atemwegs über das Nasenloch des Patienten an einem Schnittpunkt schneidet, die Fläche auf der Innenseite des Gasflow-Konus und die Fläche des Atemwegs im Nasenloch des Patienten distal zum Schnittpunkt eine Überdruckfläche definiert, wobei sich eine laminare Überdruckströmung entwickelt, die Fläche auf der Außenseite des Gasflow-Konus eine Unterdruckfläche definiert, die Umgebungsluft in den Atemweg über das Nasenloch des Patienten schleppt, die Kombination von Gas aus der Gaszufuhrquelle (407, 409) und der in den Atemweg über das Nasenloch des Patienten geschleppten Luft Atemhilfe bereitstellt.

2. System nach Anspruch 1, wobei das Verteilerstück (505) zur Positionierung der Düse (509) in einem Winkel relativ zu einer Mittellinie des Atemwegs über ein Nasenloch konfiguriert ist.

3. System nach Anspruch 1, weiter umfassend einen oder mehr Sensor(en), wobei der eine oder mehr Sensor(en) einen Sensorkanal umfasst/umfassen, der sich distal, weg von der Düse erstreckt.

4. System nach Anspruch 3, wobei der Sensorkanal konfiguriert ist, um sich während der Verwendung in den Atemweg über ein Nasenloch des Patienten zu erstrecken.

5. System nach Anspruch 1, wobei die Düse konfiguriert ist, um ein oval geformtes Flowprofil für die Gaszufuhr in dem Atemweg über das Nasenloch eines Patienten zu schaffen.

6. System nach Anspruch 1, weiter umfassend eine Strahlpumpendrossel, die einen Strömungsweg beinhaltet.

7. System nach Anspruch 1, wobei das System zur Bereitstellung eines Überdrucks von ca. 0,7 bis 34,5 mbar (0,01 bis 0,50 psi) konfiguriert ist.

8. System nach Anspruch 1, wobei das System konfiguriert ist, um die Positionierung der Düse bis zu 3,8 cm (1,5 Zoll) außerhalb des Eingangs zum Atemweg über das Nasenloch des Patienten zu erlauben.

9. System nach Anspruch 1, wobei das System konfiguriert ist, um die Synchronisierung der Gaszufuhr durch die Düse mit einem Atemmuster des Patienten zu erlauben.

10. System nach Anspruch 1, weiter umfassend ein tragbares Beatmungsgerät (401), wobei das Gas von der Gaszufuhrquelle (407, 409) durch das tragbare Beatmungsgerät (401) gesteuert wird.

11. System nach Anspruch 1, wobei die bereitgestellte Atemhilfe mechanisch zur Reduktion der Atemarbeit zur Behandlung der Ateminsuffizienz beiträgt.

## Revendications

1. Système de fourniture d'une assistance ventilatoire à un patient, le système comprenant une source de distribution de gaz (407, 409), un circuit de distribution de gaz (413), et une interface de ventilation nasale non étanche (400), l'interface de ventilation nasale non étanche (400) étant configurée pour communiquer avec la voie respiratoire de narine d'un patient tout en permettant au patient de respirer l'air ambiant directement sans qu'il ne s'écoule à travers l'interface de ventilation nasale non étanche (400), dans lequel :
l'interface de ventilation nasale non étanche (400) comprend un collecteur (505), le collecteur (505) comprenant une buse (509) pouvant être connectée à une tubulure de distribution de gaz et à la source de distribution de gaz (407, 409) et configurée pour la communication avec la voie respiratoire de narine d'un patient, le collecteur (505) étant configuré pour positionner la buse (509) à une certaine distance d'une entrée de narine de la voie respiratoire de narine du patient de sorte qu'un cône de flux de gaz défini par le gaz sortant de la buse (509) croise une paroi interne de la voie respiratoire de narine du patient au niveau d'un point d'intersection, la zone à l'intérieur du cône de flux de gaz et la zone au sein de la voie respiratoire de narine du patient distales par rapport au point d'intersection définissant une zone de pression positive dans lequel un flux de pression positive laminaire se développe, la zone à l'extérieur du cône de flux de gaz définissant une zone de pression négative entraînant de l'air ambiant jusque dans la voie respiratoire de narine du patient, la combinaison de gaz de la source de distribution de gaz (407, 409) et de l'air entraîné jusque dans la voie respiratoire de narine du patient fournissant une assistance ventilatoire.

2. Système selon la revendication 1, dans lequel le collecteur (505) est configuré pour positionner la buse (509) selon un angle par rapport à une ligne centrale d'une voie respiratoire de narine.

3. Système selon la revendication 1, comprenant en outre un ou plusieurs détecteurs, dans lequel les un ou plusieurs détecteurs comprennent un canal de détection qui s'étend de manière distale en s'éloignant de la buse.

4. Système selon la revendication 3, dans lequel le canal de détection est configuré pour s'étendre jusque dans la voie respiratoire de narine d'un patient pendant l'utilisation.

5. Système selon la revendication 1, dans lequel la buse est configurée pour créer un profil de flux de distribution de gaz de forme ovale au sein de la voie respiratoire de narine d'un patient.

6. Système selon la revendication 1, comprenant en outre un col de pompe à injection comprenant un parcours d'écoulement.

7. Système selon la revendication 1, dans lequel le système est configuré pour fournir une pression positive d'approximativement 0,7 à 34,5 mBar (0,01 à 0,50 psi).

8. Système selon la revendication 1, dans lequel le système est configuré pour permettre que la buse soit positionnée à un maximum de 3,8 cm (1,5 pouce) à l'extérieur de l'entrée de la voie respiratoire de narine du patient.

9. Système selon la revendication 1, dans lequel le système est configuré pour permettre que la distribution de gaz à travers la buse soit synchronisée avec le rythme respiratoire du patient.

10. Système selon la revendication 1, comprenant en outre un ventilateur portable (401), dans lequel le gaz de la source de distribution de gaz (407, 409) est commandé par le ventilateur portable (401).

11. Système selon la revendication 1, dans lequel l'assistance ventilatoire fournie contribue mécaniquement à la réduction du travail de respiration pour traiter l'insuffisance respiratoire.
